# EUROPEAN PATENT APPLICATION

(11) **EP 4 068 336 A1**
(43) Date of publication of application: **05.10.2022**
(21) Application number: 21177669.5
(22) Date of filing: 04.06.2021
(51) Int. Cl.: H01J 37/32, A61L 2/14, B65B 55/00, H05H 1/24

(54) **RAPID PLASMA STERILIZATION DEVICE FOR SURFACES OF FOOD PACKAGING MATERIALS**

(30) Priority: 31.03.2021 CN 202110347338
(71) Applicant: Liaoning Chunguang Pharmaceutical Equipment Corp. Ltd., Jinzhou City Liaoning (CN); Dalian University of Technology, Dalian, Liaoning 116024 (CN)
(72) Inventor: LIU, Dongping, Dalian City (CN); BI, Chunguang, Jinzhou City (CN); WANG, Xi, Dalian City (CN); WANG, Qiang, Dalian City (CN); ZHANG, Zhicheng, Jinzhou City (CN); CHEN, Jianan, Jinzhou City (CN); WU, Yang, Jinzhou City (CN); TONG, Yinling, Jinzhou City (CN); LI, Chao, Jinzhou City (CN)
(74) Representative: Bjerkén Hynell KB

(57) **Abstract**

A rapid plasma sterilization device for surfaces of food packaging materials is provided, which relates to the technical field of sterilization devices. The rapid plasma sterilization device includes two sub-modules with the same structure and arranged in a front-back direction. A gap (7) is formed between the two sub-modules. Each sub-module includes a fixed seat (1), a sealing cover (2), dielectric plates (5), and circuit boards (4). Electrodes are arranged on one side surface of each dielectric plate (5). The electrodes are electrically connected to a high-voltage power supply. Ground electrode wires (6) are arranged on the other side surface of each dielectric plate (5). The ground electrode wires (6) are electrically connected to the circuit boards (4). Both the dielectric plates (5) and the circuit boards (4) are arranged in the fixed seat (1). The sealing cover (2) is located on one side, far away from the gap (2), of the fixed seat (1), and the ground electrode wires (6) are located on one side, close to the gap (7), of the fixed seat (1). Outer surfaces of a food handle material can be subjected to disinfection and sterilization rapidly and effectively, and the sterilization efficiency is high. Raw materials required are water and air, and are clean and pollution-free. The internal air humidity can be effectively and accurately controlled by controlling the flow rate of water droplets through a peristaltic pump, so as to realize high-efficiency sterilization treatment. The structure of a plasma reaction device is simple, and facilitates installation and maintenance.

## Description

### TECHNICAL FIELD

The present disclosure relates to the technical field of sterilization devices, and in particular, to a rapid plasma sterilization device for surfaces of food packaging materials.

### BACKGROUND ART

China is a country with all kinds of food. With the development of China and increasingly mature technology and large scale of food processing industry, people's awareness and demand for health and food safety are becoming more and more intense. Most food purchased on the market is packaged products, so the problem about the safety of a surface of an outer food packaging also becomes a major problem that needs to be concerned and fully solved. Therefore, it is of great significance to sterilize the outer food packaging.

Taking cheese packaging as an example, cheese packaging includes not only a packaging material outside cheese, but also a stick handle. However, the packaging material and the stick handle may be in contact with the outside world and are possible to be in contact with viruses and bacteria to cause pollution to food during processing. Therefore, it is very important to perform disinfection and sterilization in a production process of food packaging materials.

With the development of plasma technology, the plasma technology is attracting more and more attention. In recent ten years, the sterilization technology using plasmas has set off a global upsurge. The plasma sterilization technology has a very important application value indeed. This sterilization method is environmentally friendly, low in cost, simple in equipment, and high in sterilization efficiency and secondary pollution cannot be produced after treatment. Plasma contains various activated species, including hydroxyls, ozone, and nitrogen oxides. These activated species can be used for the rapid inactivation of various microorganisms on the large surface of a cheese packaging material.

### SUMMARY

In order to solve the above-mentioned technical problems, the present disclosure provides a plasma sterilization device, which is used for rapidly sterilizing surfaces of the food packaging materials.

To achieve the above objective, the present disclosure provides the following solutions:

The present disclosure provides a rapid plasma sterilization device for surfaces of food packaging materials, including two sub-modules with same structure and arranged in a front-back direction. A gap is formed between the two sub-modules. Each sub-module includes a fixed seat, a sealing cover, dielectric plates and circuit boards. Electrodes are arranged on one side surface of each of the dielectric plates. The electrodes are electrically connected to a high-voltage power supply. Ground electrode wires are arranged on other side surface of each of the dielectric plates. Two ends of each of the ground electrode wires are respectively and electrically connected to the circuit boards. Both the dielectric plates and the circuit boards are arranged in the fixed seat. The sealing cover is located on one side, far away from the gap, of the fixed seat, and the ground electrode wires are located on one side, close to the gap, of the fixed seat.

Optionally, the electrodes are electrically connected to the high-voltage power supply through high-voltage wires.

Optionally, the electrodes are made of a metal foil.

Optionally, a voltage supplied from the high-voltage power supply is 7 to 21 kV, and a working frequency is 10 kHz.

Optionally, grooves are formed in the other side surface of each of the dielectric plates, and the ground electrode wires are arranged in the grooves.

Optionally, the dielectric plates are insulating material plates.

Optionally, the dielectric plates are ceramic plates, Printed Circuit Boards (PCBs), quartz plates, or polyimide plates.

Optionally, water tanks are arranged on two sides of the fixed seat. Water inlets and water outlets are formed in the water tanks. The water inlets are communicated with a water injection tank. The water outlets are communicated with a wastewater tank. A pump is arranged between the water injection tank and the water inlets.

Optionally, a flow velocity of water flowing in the water tanks is 20 to 50 mL/min.

Optically, multiple fans are arranged on the sealing cover.

Compared with the prior art, the present disclosure achieves the following technical effects.

Sterilization treatment is performed by reactive species in plasmas generated by surface dielectric discharge. These reactive species have very strong chemical activity and long half-life, and can biochemically react with bacteria, fungi or viruses on the surfaces of the packaging materials to kill them, destroy the integrity of their membranes and in turn their structures. The production of reactive oxygen species and reactive nitrogen species are important factors for sterilization. The ground electrode wires of the present disclosure are uniformly arranged on the surface of each dielectric plate, and the distance between every two wires is very small, which can produce large-area uniform discharge and reduce the surface temperature. Each metal wire is fixed by the PCBs that are placed parallel to the dielectric boards and coplanar with the surface of the corresponding dielectric board, and respectively located at the two ends of the metal wire, so as to ensure that the metal wire is straightened without bending. A small amount of water is introduced into the device, to provide a humid environment for a plasma generating area, in which air and water molecules are ionized to produce oxidizing species such as peroxynitrous acid, through a series of chemical reactions, thus the sterilization efficiency of the surface is improved. In addition, a large-area packaging material to be treated can be treated in the device in a moving manner, so that both front surface and rear surface can be treated.

The rapid plasma sterilization device for the surfaces of the food packaging materials of the present disclosure can rapidly and effectively perform disinfection and sterilization on outside surfaces of the food handle materials, and the disinfection and sterilization efficiency is high. The raw materials required by the plasma reaction device are water and air, and the raw materials for reaction are clean and pollution-free. The internal air humidity of the device can be effectively and accurately controlled by controlling the flow rate of water droplets serving as a raw material in the reaction device through a peristaltic pump, to realize high-efficiency sterilization treatment. The structure of the plasma reaction device is simple, and facilitates installation and maintenance.

### BRIEFT DESCRIPTION OF THE DRAWINGS

In order to describe the technical solutions in the embodiments of the present disclosure or in the prior art more clearly, the following briefly describes the accompanying drawings required for describing the embodiments. Apparently, the accompanying drawings in the following description are merely some embodiments of the present disclosure, and those of ordinary skill in the art may still derive other drawings from these accompanying drawings without creative efforts.
FIG. 1 is a schematic structural diagram of a sub-module in a rapid plasma sterilization device for surfaces of food packaging materials of the present disclosure;
FIG. 2 is a schematic diagram of an inside structure of the sub-module in the rapid plasma sterilization device for the surfaces of the food packaging materials of the present disclosure;
FIG. 3 is a schematic side diagram of a structure of the rapid plasma sterilization device for the surfaces of the food packaging materials of the present disclosure;
FIG. 4 is a schematic structural diagram of a dielectric plate in the rapid plasma sterilization device for the surfaces of the food packaging materials of the present disclosure;
FIG. 5 is a schematic structural diagram of a circuit board in the rapid plasma sterilization device for the surfaces of the food packaging materials of the present disclosure;
FIG. 6 is a schematic structural diagram of a fixed seat of the rapid plasma sterilization device for the surfaces of the food packaging materials of the present disclosure;
FIG. 7 is a graph showing experimental result data of the rapid plasma sterilization device for the surfaces of the food packaging materials of the present disclosure.

Reference signs in drawings: 1-fixed seat; 2-sealing cover; 3-fan; 4-circuit board; 5-dielectric plate; 6-ground electrode wire; 7-gap; 8-water tank; 9-guide plate fixing groove; 10-water inlet; 11-water outlet.

### DETAILED DESCRIPTION OF THE EMBODIMENTS

The technical solutions in embodiments of the present disclosure will be clearly and completely described herein below with reference to the accompanying drawings in the embodiments of the present disclosure. Apparently, the described embodiments are merely part rather than all of the embodiments of the present disclosure. On the basis of the embodiments of the present disclosure, all other embodiments obtained by those of ordinary skill in the art without creative work fall within the scope of protection of the present disclosure.

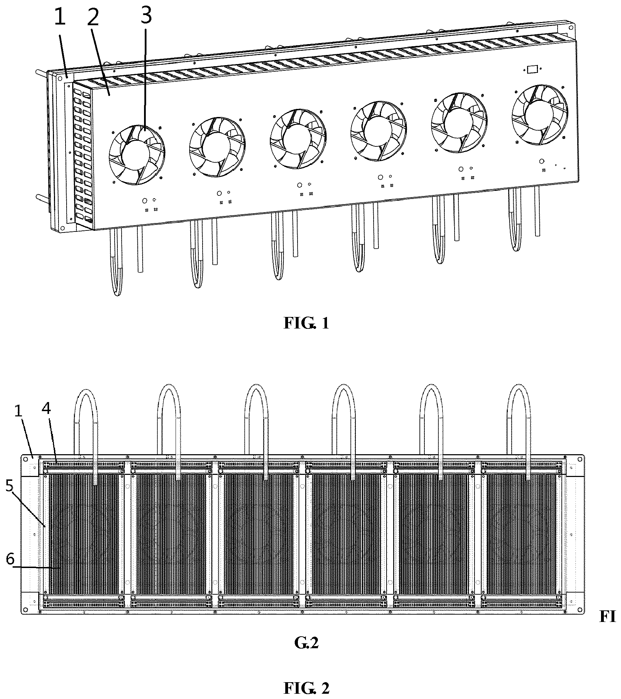

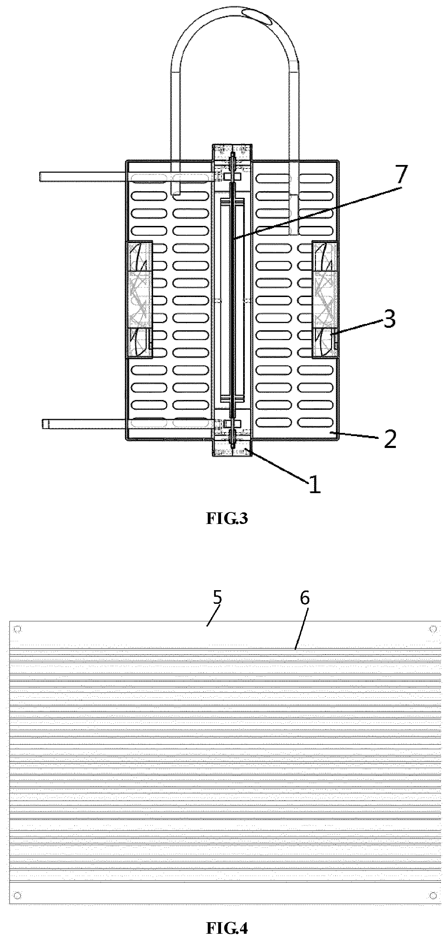

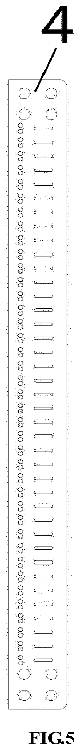

As shown in FIG. 1 to FIG. 7, the present embodiment provides a rapid plasma sterilization device for surfaces of food packaging materials, including two sub-modules with the same structure and arranged in a front-back direction. A gap 7 is formed between the two sub-modules. Each sub-module includes a fixed seat 1, a sealing cover 2, dielectric plates 5, and circuit boards 4. Each dielectric plate 5 is provided on one side surface thereof with electrodes, which are electrically connected to a high-voltage power supply. Ground electrode wires 6 are arranged on the other side surface of the dielectric plate 5. Two ends of each ground electrode wire 6 are respectively and electrically connected to the two circuit boards 4, which are arranged opposite to each other and have the corresponding dielectric plate 5 sandwiched therebetween. Both the dielectric plates 5 and the circuit boards 4 are arranged in the fixed seat 1. The sealing cover 2 is located far away from the gap 7, at one side of the fixed seat 1; and the ground electrode wires 6 are located close to the gap 7, at the one side of the fixed seat 1.

In the present specific embodiment, the electrodes are electrically connected to the high-voltage power supply through high-voltage wires. The electrodes are made of a copper foil. The voltage of the high-voltage power supply is 7 to 21 kV, and the working frequency is 10 kHz.

Grooves are formed in the other side surface of each dielectric plate 5, and the ground electrode wires 6 are arranged in the grooves. The dielectric plates 5 are made of ceramics, and each have a length of 340 mm to 380 mm, a width of 180 mm to 220 mm, and a thickness of 2 mm. The grooves formed in each dielectric plates 5 each have a depth of 0.3 mm and a width of 0.6 mm, and the distance between every two grooves is 4 mm. The ground electrode wires 6 are filamentous or mesh-like metal such as steel, tungsten or silver, and are embedded into the grooves of the dielectric plates 5.

The dielectric plates 5 are ceramic plates.

Water tanks 8 are arranged on two sides of the fixed seat 1. Water inlets 10 and water outlets 11 are formed in the water tanks 8. The water inlets 10 are communicated with a water injection tank. The water outlets 11 are communicated with a wastewater tank. A peristaltic pump is arranged between the water inlets 10 and the water injection tank. A flow velocity of water bumped into the water injection tank from the water tanks 8 are 20 to 50 mL/min.

Multiple fans 3 are arranged on the sealing cover 2. The fans 3 can be arranged on two sides of the sealing cover 2, and may also be located on end surfaces of the sealing cover 2. The fans 3 are fixed to the sealing cover 2 through stainless steel or aluminum alloy brackets.

The sealing cover 2 and the fixed seat 1 are made of aluminum alloy.

During operation, a material to be treated falls freely by gravity from top to bottom, the supplied voltage from the power supply is 7 to 21 kV, and the discharge frequency is 10 kHz, then plasmas are generated at the ground electrode wires 6 of the dielectric plate 5. A temperature of a space in a plasma reaction device is high, so that water droplets in the water tanks 8 volatilize to form a humid air environment. In such humid air environment, the plasmas generated by discharging can rapidly perform disinfection and sterilization on the surfaces of a handle material in a short time. In addition, during the operation of the device, a user needs to connect the fans to the power supply, so as to realize force air cooling of the high-voltage electrodes and the dielectric plate 5.

The following describes the rapid plasma sterilization device for the surfaces of the food packaging materials in the present embodiment in detail, in combination with the accompanying drawings and more specific embodiments.

### Embodiment 1:

A rapid plasma sterilization method for surfaces of a cheese packaging material includes the following specific process.

High-voltage wires led out from the device are connected to the high-voltage power supply, and the device is grounded. The water inside the water injection tank flows into the device through water pipes, and the flow velocity of the water entering the device is controlled through the peristaltic pump. The fans 3 rotate normally, and the high-voltage power supply is turned on to warm up the device for 1 minute. A packaging material to be treated is fed into the device from an upper side of the device, passing through the tiny gap 7 formed between the fixed seats 1. The packaging material is dragged by an automatic machine to allow front and back surfaces of the material to be treated. The treatment will ends after 10 seconds, and then the material is fed out from an outlet at a bottom of the device.

### Embodiment 2:

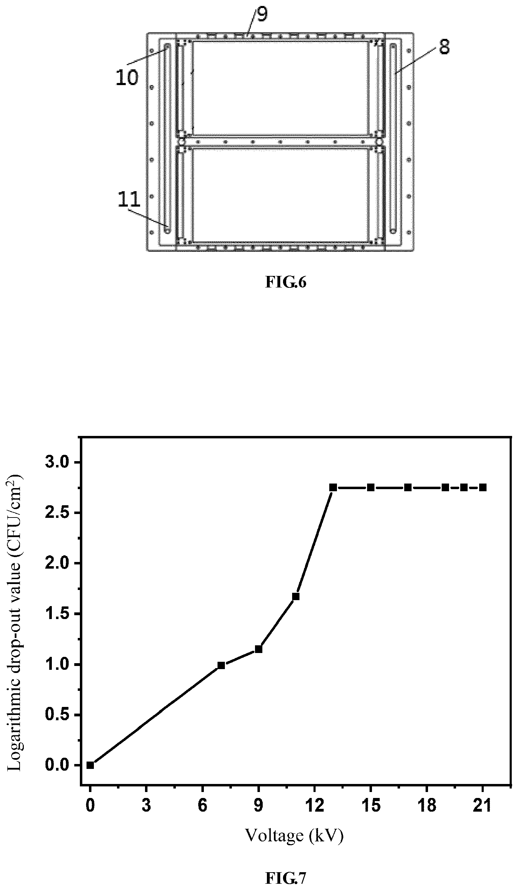

The prepared ten groups of 1 × 1 cm² bacterial tablets containing *Escherichia coli* are arranged on the ground electrode wires 6 to be in direct contact with the plasmas for treatment. The first group of bacterial tablets is treated with a supplied voltage of 7 kV; the second group of bacterial tablets is treated with a supplied voltage of 9 kV; the third group of bacterial tablets is treated with a supplied voltage of 11 kV; the fourth group of bacterial tablets is treated with a supplied voltage of 13 kV; the fifth group of bacterial tablets is treated with a supplied voltage of 15 kV; the sixth group of bacterial tablets is treated with the supplied voltage of 17 kV; the seventh group of bacterial tablets is treated with the supplied voltage of 19 kV; the eighth group of bacterial tablets is treated with the supplied voltage of 20 kV; the ninth group of bacterial tablets is treated with the supplied voltage of 21 kV; the tenth group of bacterial tablets serves as a comparison group without any treatment. Each group of bacterial tablets is taken out after being treated by plasmas for 10 seconds, and is placed in a centrifugal tube containing 2 mL sterile water for flushing the bacteria from the bacterial tablets. Finally, 300 µL liquid is taken out from 2 mL bacteria solution for each group and a culture medium is coated with the liquid. The culture medium is placed in an incubator for being cultured overnight, and then is observed. As shown in FIG. 7, when the supplied voltage is 13 kV, the logarithmic decrement value of the quantity of the bacteria contained on the bacterial tablets after being subject to the treatment by the plasmas, reaches the maximum. It can be seen that in a humid air, the plasmas generated by the discharge along a dielectric surface can effectively inactivate surface bacteria in a short time. In addition, the effect becomes more distinguished, as the applied voltage increases.

It should be noted that for those skilled in the art, it is obvious that the present disclosure is not limited to the details of the above exemplary embodiments, and can be implemented in other specific forms without departing from the spirit or basic features of the present disclosure. Therefore, from any point of view, the embodiments should be regarded as exemplary but not restrictive. The scope of the present disclosure is limited by the attached claims rather than the above description. Therefore, it is intended to include all changes within the meaning and scope of the equivalent elements of the claims in the present disclosure, and any reference numeral in the claims shall not be regarded as limiting the claims involved.

Specific examples are applied in the specification to illustrate the principle and implementation mode of the present disclosure. The description of the above examples is only used to help understand the method and core idea of the present disclosure. Meanwhile, for those of ordinary skill in the art, according to the idea of the present disclosure, there will be changes in the specific implementation mode and application scope. In conclusion, the content of the present disclosure shall not be construed as a limitation to the present disclosure. In conclusion, in the embodiments of the present disclosure, the present disclosure provides the following technical solutions:
Solution 1: A rapid plasma sterilization device for surfaces of food packaging materials, comprising two sub-modules with same structure and arranged in a front-back direction, wherein a gap is formed between the two sub-modules; the sub-modules each include a fixed seat, a sealing cover, dielectric plates, and circuit boards; electrodes are arranged on one side surface of each of the dielectric plates; the electrodes are electrically connected to a high-voltage power supply; ground electrode wires are arranged on other side surface of each of the dielectric plates; two ends of each of the ground electrode wires are respectively and electrically connected to the circuit boards; both the dielectric plates and the circuit boards are arranged in the fixed seat; the sealing cover is located on one side, far away from the gap, of the fixed seat and the ground electrode wires are located on other side, close to the gap, of the fixed seat.
Solution 2: The rapid plasma sterilization device for the surfaces of the food packaging materials according to solution 1, wherein the electrodes are electrically connected to the high-voltage power supply through high-voltage wires.
Solution 3: The rapid plasma sterilization device for the surfaces of the food packaging materials according to any one of the previous solutions, wherein the electrodes are made of a metal foil.
Solution 4: The rapid plasma sterilization device for the surfaces of the food packaging materials according to any one of the previous solutions, wherein a voltage supplied from the high-voltage power supply is 7 to 21 kV, and a working frequency is 10 kHz.
Solution 5: The rapid plasma sterilization device for the surfaces of the food packaging materials according to any one of the previous solutions, wherein grooves are formed in the other side surface of each of the dielectric plates, and the ground electrode wires are arranged in the grooves.
Solution 6: The rapid plasma sterilization device for the surfaces of the food packaging materials according to any one of the previous solutions, wherein the dielectric plates are insulating material plates.
Solution 7: The rapid plasma sterilization device for the surfaces of the food packaging materials according to any one of the previous solutions, wherein the dielectric plates are ceramic plates, Printed Circuit Boards (PCBs), quartz plates, or polyimide plates.
Solution 8: The rapid plasma sterilization device for the surfaces of the food packaging materials according to any one of the previous solutions, wherein water tanks are arranged on two sides of the fixed seat; water inlets and water outlets are formed in the water tanks; the water inlets are communicated with a water injection tank; the water outlets are communicated with a wastewater tank; a pump is arranged between the water injection tank and the water inlets.
Solution 9: The rapid plasma sterilization device for the surfaces of the food packaging materials according to any one of the previous solutions, wherein a flow velocity of water flowing in the water tanks is 20 to 50 mL/min.
Solution 10: The rapid plasma sterilization device for the surfaces of the food packaging materials according to any one of the previous solutions, wherein a plurality of fans are arranged on the sealing cover.

## Claims

1. A rapid plasma sterilization device for surfaces of food packaging materials, comprising two sub-modules with same structure and arranged in a front-back direction, wherein a gap (7) is formed between the two sub-modules; the sub-modules each include a fixed seat (1), a sealing cover (2), dielectric plates (5), and circuit boards (4); electrodes are arranged on one side surface of each of the dielectric plates (5); the electrodes are electrically connected to a high-voltage power supply; ground electrode wires (6) are arranged on other side surface of each of the dielectric plates (5); two ends of each of the ground electrode wires (6) are respectively and electrically connected to the circuit boards (4); both the dielectric plates (6) and the circuit boards (4) are arranged in the fixed seat; the sealing cover (2) is located on one side, far away from the gap (7), of the fixed seat (1) and the ground electrode wires (6) are located on other side, close to the gap (7), of the fixed seat (1).

2. The rapid plasma sterilization device for the surfaces of the food packaging materials according to claim 1, wherein the electrodes are electrically connected to the high-voltage power supply through high-voltage wires.

3. The rapid plasma sterilization device for the surfaces of the food packaging materials according to claim 1, wherein the electrodes are made of a metal foil.

4. The rapid plasma sterilization device for the surfaces of the food packaging materials according to claim 1, wherein a voltage supplied from the high-voltage power supply is 7 to 21 kV, and a working frequency is 10 kHz.

5. The rapid plasma sterilization device for the surfaces of the food packaging materials according to claim 1, wherein grooves are formed in the other side surface of each of the dielectric plates, and the ground electrode wires (6) are arranged in the grooves.

6. The rapid plasma sterilization device for the surfaces of the food packaging materials according to claim 1, wherein the dielectric plates are insulating material plates.

7. The rapid plasma sterilization device for the surfaces of the food packaging materials according to claim 1, wherein the dielectric plates (5) are ceramic plates, Printed Circuit Boards (PCBs), quartz plates, or polyimide plates.

8. The rapid plasma sterilization device for the surfaces of the food packaging materials according to claim 1, wherein water tanks (8) are arranged on two sides of the fixed seat (1); water inlets (10) and water outlets (11) are formed in the water tanks (8); the water inlets (10) are communicated with a water injection tank; the water outlets (11) are communicated with a wastewater tank; a pump is arranged between the water injection tank and the water inlets (10).

9. The rapid plasma sterilization device for the surfaces of the food packaging materials according to claim 8, wherein a flow velocity of water flowing in the water tanks (8) is 20 to 50 mL/min.

10. The rapid plasma sterilization device for the surfaces of the food packaging materials according to claim 1, wherein a plurality of fans (3) are arranged on the sealing cover (2).
